# EUROPEAN PATENT SPECIFICATION

(11) **EP 2 436 383 B1**
(45) Date of publication and mention of the grant of the patent: **19.07.2017**
(21) Application number: 10780650.7
(22) Date of filing: 28.05.2010
(51) Int. Cl.: A61K 31/167, A61K 9/48, A61K 31/34, A61K 47/34, A61P 1/02, A61P 25/02, A61K 9/50

(54) **ORAL MEDICINAL COMPOSITION AND ORAL MEDICINAL CAPSULE HAVING THE COMPOSITION ENCAPSULATED THEREIN**
ORAL EINNEHMBARE MEDIZINISCHE ZUSAMMENSETZUNG UND ORAL EINNEHMBARE KAPSEL MIT DER DARIN EINGEKAPSELTEN ZUSAMMENSETZUNG
COMPOSITION MÉDICINALE ORALE ET CAPSULE MÉDICINALE ORALE CONTENANT UNE COMPOSITION ENCAPSULÉE À L'INTÉRIEUR

(30) Priority: 29.05.2009 JP 2009130917
(43) Date of publication of application: 04.04.2012
(73) Proprietor: Morishita Jintan Co., Ltd., Osaka-shi Osaka 540-8566 (JP)
(72) Inventor: SHIMOO, Tsuyoshi, Osaka-shi Osaka 540-8566 (JP)
(74) Representative: dompatent von Kreisler Selting Werner - Partnerschaft von Patent- und Rechtsanwälten mbB
(86) International application number: PCT/JP2010/059103
(87) International publication number: WO 2010/137696

(56) References cited:
- EP-A2- 0 215 635
- EP-A2- 0 525 731
- JP-A- 1 272 521
- JP-A- 3 066 612
- JP-A- 3 501 737
- JP-A- 5 031 352
- JP-A- 9 151 127
- JP-A- 62 116 508
- JP-A- 62 265 235
- JP-A- 63 060 924
- JP-A- 2001 238 611
- JP-A- 2005 015 479
- JP-T- 2003 525 895
- JP-T- 2006 521 292
- US-A1- 2003 185 761
- US-A1- 2004 202 698
- US-A1- 2006 018 965
- RYOSEI KAMAGUCHI: "Taso Seamless Capsule no Shokuhin eno Oyo", FOOD STYLE 21, XX, JP, vol. 13, no. 3, 1 March 2009 (2009-03-01), pages 74-75, XP008161813, ISSN: 1343-9502

## Description

### TECHNICAL FIELD

The present invention relates to an oral seamless capsule.

### BACKGROUND ART

In dental care, reductions of pain in needle insertion for anesthetic injection at the time of tooth extraction, pain in removing dental plaque, pain in scraping ceca, and the like are an extremely important object to be achieved for relieving emotional distress of patients and performing medical practice easily. Conventionally, ointments, jellies, sprays, tablets, troches, granules, and the like containing anesthetics have been used a lot for anesthesia and preliminary anesthesia.

The above-described medical agents for anesthesia can be obtained by known methods in the cases of ointments, jellies, and sprays. However, in these preparation forms, it is difficult to apply (or administer) them quantitatively to the inside of the mouth and also there are problems with their handling.

The above-described medical agents for anesthesia can be obtained by mixing an anesthetic into a vehicle, a lubricant, or the like, followed by molding in the cases of a tablet, troche, or granule form. When these medical agent forms are used as medical agents for anesthesia, there are problems to be solved, such as 1) that stimulation is given to the mouth and displeased feeling is given due to sharpening in licking them during their release in the mouth, and 2) that a powdery anesthetic exhibits low solubility in the mouth to take time for its elution. Thus, remedy of these problems has been demanded.

In order to solve the above-described problems, some methods have been proposed. JP 2004-520410 A1 (Patent Document 1) discloses an oral controlled-release preparation containing a medical agent containment matrix that is insoluble in the mouth of a patient, and a central nervous system-affecting medical agent incorporated into the insoluble matrix. However, it is impossible to release above-described displeased feeling that accompanies the tablets or troches, since this oral controlled-release preparation has a solid dosage form.

In the liquid preparation prepared in Examples of JP 2001-10977 A1 (Patent Document 2), sodium citrate is added together with a local anesthetic, and a liquid preparation to be used as a gargle in the mouth or at the pharyngeal part is disclosed. However, the content of the local anesthetic contained in such an oral liquid formulation is only 0.05 to 0.3%, and it is unlikely that such a small content can reduce the pain from inflammation of the oral mucosa satisfactorily as an effective ingredient.
EP 0215635A2 (Patent Document 3) describes chewable hard gelatin capsules for buccal activity, the carrier being PEG having a MW of 200-10000, wherein the active ingredient, which can be a cardioactive or an analgesic drug is present in amounts of up to 20% of the composition. Further components are vegetable and animal oils and fats.
Further, EP 523 731 (Patent Document 4) describes seamless capsules comprising a herb extract, PEG 400 and further auxiliary agents.

### PRIOR ART DOCUMENTS

### PATENT DOCUMENTS

Patent Document 1: JP 2004-520410 A1
Patent Document 2: JP 2001-10977 A1
Patent Document 3: EP 0215635 A2
Patent Document 4: EP 0525731 A2

### SUMMARY OF THE INVENTION

### PROBLEMS TO BE SOLVED BY THE INVENTION

The present invention solves the above-described drawbacks and intends to give no stimulation or no displeased feeling in the course of dissolution in a mouth, improve the solubility of a medical agent to be exhibited in a mouth, and improve the absorbability of a medical agent in a mouth.

### SOLUTIONS TO THE PROBLEMS

That is, the present invention provides an oral seamless capsule comprising a capsule film, a content enclosed in the film, and an intermediate layer intervening between the content and the capsule film, wherein the content is an oral drug composition comprising
(a) a drug being a local anesthetic that is low in solubility in both water and oil, but is soluble in a polyethylene glycol,
(b) a polyethylene glycol that is solid at temperatures of 40°C or lower,
(c) an oily or aqueous auxiliary agent, and
wherein the intermediate layer is made of fat having a melting point of 40°C or lower.

The auxiliary agent (c) may preferably be a freshener, an anti-inflammatory agent, a local anesthetic, or a mixture thereof.

The medical agent (a) may preferably be contained in an amount of 0.01 to 40% by weight in the oral medical composition.

Preferably, the seamless capsule is composed of a capsule film, a content encapsulated in the capsule film, and an intermediate layer intervening between the content and the capsule film, wherein the content is the oral medical composition of the present invention and the intermediate layer is a fat having a melting point of 40°C or lower.

### EFFECTS OF THE INVENTION

In the present invention, a medical agent that is low in solubility in both water and oils but is soluble in a polyethylene glycol is dissolved in a polyethylene glycol that is solid at temperatures of 40°C or lower. It becomes possible to uniformly disperse an aqueous or oily auxiliary agent and exhibit an aqueous and oily uniform elution behavior in the oral cavity, in other words, exhibit controlled release of a medical agent, so that it can achieve to control the elution property of a medical agent in the mouth. Moreover, the oral medical composition of the present invention can be molded into any shape. In addition, it is molded into a solid form with a polyethylene glycol without using a conventional solid vehicle or the like, so that its dissolution in the oral cavity proceeds smoothly and it hardly physically stimulates the inside of the oral cavity.

If the oral medical composition of the present invention is covered with a seamless capsule to be formed into an oral medical capsule, it dissolves in the oral cavity more gently and stimulation in the oral cavity is also remedied greatly. Moreover, the film of the seamless capsule dissolves immediately in the oral cavity, so that the dissolution of the oral medical composition starts and the medical agent is eluted slowly through the above-described smooth and gentle dissolution, thereby demonstrating its efficacy under optimum conditions.

### MODE(S) FOR CARRYING OUT OF THE INVENTION

The oral medical composition of the present invention contains
(a) a medical agent that is low in solubility in both water and oils but is soluble in a polyethylene glycol,
(b) a polyethylene glycol that is solid at temperatures of 40°C or lower, and
(c) an oily or aqueous auxiliary agent.
The respective components are described below.

### Component (a)

Component (a) of the present invention is a medical agent that is low in solubility in both water and oils but is soluble in a polyethylene glycol. Such medical agents are not particularly restricted and may be any medical agents that can be used through dissolution in the mouth. More specific examples of the medical agent include, for example, local anesthetics, blood circulation improvers, and the like that exhibit low absorbability in the intestinal tract. Such local anesthetics may preferably include at least one member selected from the group consisting of cocaine, procaine, chloroprocaine, tetracaine, benzocaine, lidocaine, mepivacaine, prilocaine, bupivacaine, dibucaine, propoxycaine, etidocaine, dyclonine, oxybuprocaine, tecaine, amethocaine, propitocaine, piperocaine, quatacaine, butanicaine, hexothiocaine, meprylcaine, epirocain, amylocaine, isobucaine, tricaine, parethoxycaine, pyrrocaine, hexylcaine, metabutoxycaine, xylocaine, metabutethamine, oxethazaine, pyridoxine, bromoxine, dimethisoquin, ethyl aminobenzoate, ethyl piperidinoacetylaminobenzoate, benzyl alcohol, chlorobutanol, and pharmaceutically acceptable salts thereof. Examples of the blood circulation improvers include glycerol trinitrate, isosorbide dinitrate, and dihydroergotoxine mesylate.

Component (a) may preferably be contained in the oral medical composition of the present invention in an amount of 0.001 to 50% by weight, preferably 0.01 to 40% by weight. If the amount is less than 0.01% by weight, the efficacy of the medical agent may not be exhibited. If the amount is 50% by weight or more, Component (a) may not dissolve completely in a polyethylene glycol and there are drawbacks such as those effects of the present invention may not be demonstrated.

### Component (b)

Component (b) of the present invention is a polyethylene glycol that is solid at temperature of 40°C or lower. Such a polyethylene glycol is represented by the following formula (1):

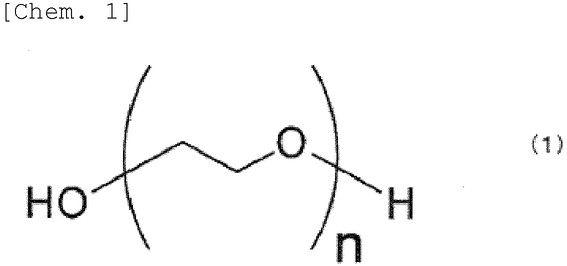

In the above chemical formula, n represents an integer of 2 to 150. The polyethylene glycols corresponding to the above formula specifically include, for example, diethylene glycol, triethylene glycol, and tetraethylene glycol; and their mixtures are also included. Polyethylene glycols are sold in the market and examples thereof include PEG-200, PEG-300, PEG-400, PEG-600, PEG-2000, PEG-3000, and PEG-6000, which are commercially available from, for example, NOF Corporation.

The polyethylene glycol of Component (b) may be contained in the oral medical composition of the present invention in an amount of 1 to 95% by weight, preferably 10 to 90% by weight. If the amount is less than 1% by weight, the efficacy of the medical agent of Component (a) may not be exhibited. If the amount is more than 95% by weight, there are drawbacks such as that a medical agent may not be encapsulated.

### Component (c)

Component (c) which is used for the oral medical composition of the present invention is an oily or aqueous auxiliary agent. In the present specification, the "auxiliary agent" is an agent that aids the medical agent of Component (a) or an agent that has an activity different from that of Component (a) and enhances or complements the efficacy of Component (a). Specific examples of the auxiliary agent include taste substances that improve taste, anti-inflammatory agents, and local anesthetics that fail to satisfy the requirements of Component (a) (e.g., water-soluble or oil-soluble local anesthetics). Examples of oily tasting components include menthol, camphor, mentha oil, eucalyptus oil, and their mixtures. Examples of oily local anesthetics include clove oil. Examples of oily anti-inflammatory agents include cinnamon oil. Examples of aqueous auxiliary agents include anti-inflammatory agents such as dipotassium glycyrrhizinate, glycyrrhetinic acid, and allantoin.

In the use of an oily auxiliary agent (c), it is possible to appropriately use a surfactant and mix it into an oral medical composition of the present invention, and the surfactant to be used in such a case can be mixed in the oral medical composition in an amount up to 20% by weight. Addition in an amount of 20% by weight or more is undesirable because it may be difficult to form a capsule in forming a capsule. The surfactant is not particularly restricted specifically and may, for example, be an anionic surfactant, a cationic surfactant, a nonionic surfactant, or an ampholytic surfactant; examples thereof include sodium lauryl sulfate, lauryl sulfate triethanolamine, lauryl sulfate ammonium, sodium dodecyl benzenesulfonate, sodium stearate, sodium salt of partially hydrogenated tallow fatty acid, potassium salt of partially hydrogenated tallow fatty acid, potassium oleate, potassium salt of castor oil, sodium alkylnaphthalenesulfonate, sodium dialkylsulfosuccinate, sodium alkyl diphenyl ether disulfonate, alkylphosphoric acid diethanolamine, potassium alkylphosphate, sodium polyoxyethylenealkylsulfate, triethanolamine polyoxyethylene alkyl ether sulfate, sodium polyoxyethylene alkyl phenyl ether sulfate, lauryltrimethylammonium chloride, stearyltrimethylammonium chloride, cetyltrimethylammonium chloride, distearyldimethylbenzylammonium chloride, alkylbenzene dimethylammonium chloride, stearylamine oleate, stearylamine acetate, stearylamine, glycerol fatty acid esters, propylene glycol fatty acid esters, sorbitan fatty acid esters, polyoxyethylene sorbitan fatty acid esters, propylene fatty acid esters, glycerol fatty acid esters, sucrose fatty acid esters, polyoxyethylene sorbitol fatty acid esters, polyoxyethylene sorbitol tetraoleate, polyoxyethylene alkyl ether, polyoxypropylene alkyl ether, polyoxyethylene polyoxypropylene glycol, polyoxyethylene polyoxypropylene alkyl ether, polyethylene glycol fatty acid ester, polyoxyethylene castor oil, polyoxyethylene hydrogenated castor oil, alkyl dimethylaminoacetate betaine, alkyldimethylamine oxide, alkyl carboxymethyl hydroxyethyl imidazolium betaine, lecithin, laurylaminopropionic acid, alkyldiaminoethylglycine, gum arabic, sodium caseinate, and powdered tragacanth. Examples of an antiseptic agent include methyl p-hydroxybenzoate, ethyl p-hydroxybenzoate, sorbic acid, phenol, cresol, and chlorocresol.

The auxiliary agent (c) may be contained in the oral medical composition of the present invention in an amount of 0.001 to 60% by weight, preferably 0.01 to 40% by weight. If the amount is less than 0.01% by weight, the function of the auxiliary agent of Component (c) may not be exhibited. If the amount is more than 60% by weight, there is a drawback, for example, that Component (a) or (b) may not be contained in an effective amount.

### Other components

The oral medical composition of the present invention may optionally contain various additives to be used for the production of general preparations in appropriate amounts. Examples of such additives include, for example, an acidulant, a foaming agent, an artificial sweetener, a perfume, a colorant, a stabilizer, a thickener, a pH adjuster, and an antiseptic agent. Examples of the thickener and the acidulant include, for example, citric acid, tartaric acid, malic acid, and ascorbic acid.

Examples of the foaming agent include, for example, sodium hydrogencarbonate and sodium carbonate. Examples of the sweetener include, for example, saccharin sodium, glycyrrhizin dipotassium, aspartame, stevia, thaumatin, and acesulfame potassium. Examples of the perfume include, for example, lemon oil, orange oil, and menthol.

The colorant is not particularly restricted as long as it is a conventionally known colorant. Examples of the colorant include, for example, natural colorants and synthetic colorants. The amount of a colorant to be used, expressed by the solid content, may be 0.001 to 5% by weight, preferably 0.01 to 1% by weight, relative to the weight amounts of the oral medical composition. Examples of the stabilizer include, for example, disodium edetate, tocopherol, and cyclodextrin.

Examples of the thickener include, for example, silica gel, alumina, (chemically modified) starch, cellulose, methylcellulose, hydroxyethylcellulose, and sodium carboxylmethylcellulose.

Examples of the pH adjuster include, for example, salts of citric acid, salts of phosphoric acid, salts of carbonic acid, salts of tartaric acid, salts of fumaric acid, salts of acetic acid, and salts of amino acids.

Such other components may be contained in the oral medical composition of the present invention in an amount of 0.001 to 60% by weight, preferably 0.1 to 40% by weight. If the amount is less than 0.01% by weight, the function of the component incorporated may not be exhibited. If the amount is more than 60% by weight, there is a drawback, for example, that Component (a) or (b) may not be contained in an effective amount.

### Oral medical composition

The oral medical composition of the present invention is obtained by mixing the above-described Components (a) through (c) and optional additives. Practically, the composition is obtained by heating the polyethylene glycol (b) to 40°C or higher to melt, then adding the medical agent of Component (a) to dissolve, then mixing Component (c) and other additives, followed by mixing, and then molding the mixture in a prescribed mold, followed by cooling.

### Oral medical capsule

The present invention is characterized by further processing the above-described oral medical composition into a capsule, especially a seamless capsule. As to the seamless capsule, generally, a content solution and a film solution are extruded through a concentric nozzle, that is, the content solution is extruded through the inner nozzle and the film solution is extruded through the outer nozzle. Then, both the solutions are dropped together into a coagulating solution to form a spherical form by surface tension, and then dried to form a seamless capsule. The method is generally called as "instillation method".

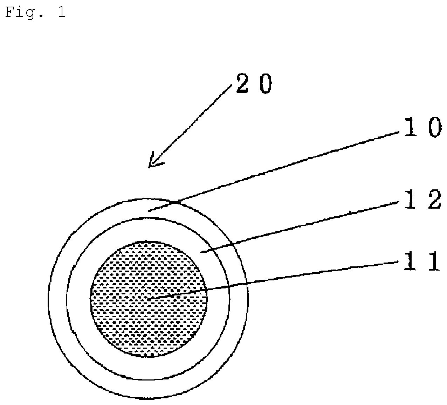

Since the oral medical capsule of the present application includes the content containing the polyethylene glycol (b) as a medium as described above, the capsule film may be degraded due to the relation between the content and the film, so that the capsule may not be stored for a long term. In such a case, an intermediate layer may be formed between the content and the film. A capsule with such an intermediate layer is illustrated in Fig. 1. Fig. 1 is a schematic sectional view illustrating an embodiment of a oral medical capsule (20) of the present invention. In Fig. 1, 10 represents a capsule film, 11 represents a capsule content, namely, an oral medical composition of the present invention, and 12 represents the intermediate layer. The fat which is used for the intermediate layer may preferably be used with selection or combination from the following so that its melting point may become 10 to 40°C. The intermediate layer includes various types of oils and fats, fatty acids, fatty acid esters of sugars, specifically, soybean oil, sesame oil, palm oil, corn oil, cotton seed oil, coconut oil, rapeseed oil, cacao butter, beef tallow, lard, horse oil, whale oil, hydrogenated oils and fats having a melting point of 40°C or lower, margarine, shortening, glycerol fatty acid ester, and sucrose fatty acid ester.

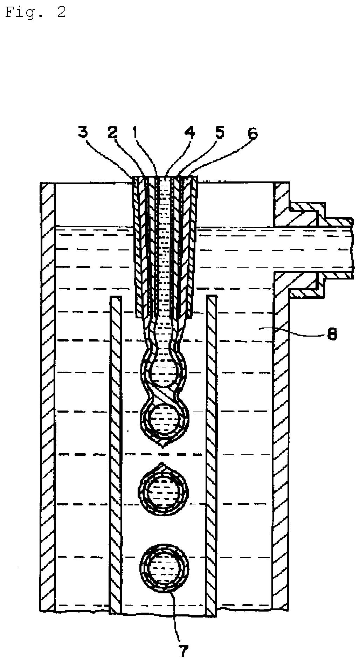

A production method in case that an intermediate layer 12 is present in a method for preparing the above-described oral medical capsule of the present invention is described briefly in reference to Fig. 2. Fig. 2 is a schematic vertical sectional view illustrating one embodiment of a nozzle part of a manufacturing apparatus suitable for producing a seamless capsule formulation by the present invention.

In Fig. 2, an oral medical composition 4 of the present invention as a content is fed into the nozzle part, and is extruded through an inner nozzle (first nozzle) 1, and an intermediate layer solution 5 is extruded through a circular hole tip of an intermediate nozzle (second nozzle) 2, respectively, and simultaneously a capsule film solution 6 is extruded through a circular hole tip of an outer nozzle (third nozzle) 3. Then, the three-phase composite jet is discharged into a cooling liquid 8, so that an oral medical capsule 7 containing the oral medical composition of the present invention as a content is obtained.

Examples of the base material to form the capsule film of the oral medical capsule of the present invention include a mixture of a protein and a water-soluble polyhydric alcohol, a mixture of a protein, a water-soluble polyhydric alcohol, and a polysaccharide, and a mixture of a polysaccharide and a water-soluble polyhydric alcohol. Examples of the protein include, for example, gelatin and collagen. Examples of the water-soluble polyhydric alcohol include, for example, sorbitol, mannitol, glycerol, propylene glycol, and polyethylene glycol. Examples of the polysaccharide include, for example, agar, gellan gum, xanthan gum, locust bean gum, pectin, salts of alginic acid, carrageenan, gum arabic, (modified) dextrin, (chemically modified) starch, pullulan, and salts of carboxymethylcellulose. When a salt of alginic acid, gellan gum, pectin, or carrageenan is used, a salt of alkali metal or a salt of alkaline earth metal may be added appropriately.

From the viewpoint of solubility adjustment of a capsule film, cross-linking treatment or coating may optionally be applied to the capsule film. When performing cross-linking treatment of a film containing a protein, it is performed by preparing a wet capsule, then washing the capsule fully with water, adding the capsule into an aqueous solution containing a cross-linking agent, and cross-linking a film surface. As the cross-linking agent, conventionally known agents can be used, and examples thereof include, for example, formaldehyde, acetaldehyde, propionaldehyde, glyoxal, glutaraldehyde, cinnamaldehyde, vanillyl aldehyde, acetone, ethyl methyl ketone, ethylene oxide, propylene oxide, potassium alum, ammonium alum, and chromium alum. The amount of the cross-linking agent to be used and the time for which the agent is caused to act vary depending on the type of the cross-linking agent. Concretely, resulting capsules are added to an aqueous solution of an amount of 50 to 100 times of the weight of the capsules, the solution containing 0.1 to 10%, preferably 0.5 to 2%, of a cross-linking agent, and are stirred for 10 to 300 seconds, thereby applying coating treatment. After cross-linking of the film, the aqueous solution containing the cross-linking agent is removed by fully washing with water, and then the water contained in the capsule film is dried, so that a seamless capsule of the present invention is obtained.

It is also permissible to perform cross-linking by enzymatic treatment with transglutaminase or the like as cross-linking curing treatment of a capsule film containing a protein using the above-described compound. In this case, the resulting capsules are added to an aqueous solution of an amount of 50 to 100 times of the weight of the capsules, the solution containing 0.1 to 10%, preferably 0.5 to 2%, of a cross-linking agent, and are stirred for 1 to 300 seconds, thereby applying coating treatment, and followed by washing with water and drying as described above, so that a seamless capsule of the present invention is obtained. When performing coating treatment, a seamless capsule of the present invention can be obtained by a method in which wet capsules are dried and then seamless capsules may be coated by an ordinary method using shellac, ethylcellulose, hydroxypropylmethylcellulose, hydroxypropylcellulose, polyvinyl pyrrolidone, cellulose TC-5, a vinylpyrrolidone-vinyl acetate copolymer, zein, ethylene wax, or the like as a base material, and castor oil, rapeseed oil, carnauba wax, cera flava, dibutyl phthalate, polyethylene glycol, glycerol, stearic acid, an ester of a fatty acid, sorbitan palmitate, polyoxyethylene stearate, acetylated monoglyceride, or the like as a plasticizer.

The average particle diameter of the oral medical capsule of the present invention may preferably be 1 to 10 mm, more preferably 2 to 8 mm. If the average particle diameter is smaller than 1 mm, its amount contained becomes small and there may be some problems, such as that the medical agent is difficult to be absorbed transdermally and that the absorption rate is excessively high.

### EXAMPLES

The present invention is illustrated in more detail below with reference to concrete examples. In the following, "part(s)" means "part(s) by weight."

### Example 1

In advance, 1 part by weight of lidocaine was dissolved in 100 parts by weight of Polyethylene glycol 600 (PEG600) under a condition of 50°C. A solution prepared by dissolving 0.3 parts by weight of sucrose fatty acid ester in 5 parts by weight of peppermint oil was mixed thereto, so that a filling solution of an innermost layer was prepared. On the other hand, a solution prepared by dissolving 100 parts by weight of gelatin and 25 parts by weight of glycerol in 800 parts by weight of distilled water under a condition of 60°C was used as a capsule film solution. Moreover, a melt prepared by melting a fatty acid triglyceride having a melting point of 25°C under a condition of 50°C was prepared as an oil to be provided between the film and the innermost layer (i.e., intermediate layer).

Then, in a capsule production machine, triple structure capsules were produced continuously by extruding the above-described filling solution through the inner nozzle of a concentric triple nozzle, the oil through the intermediate nozzle, and the above-described capsule film solution (capsule film forming material) through the outer nozzle, through their circular hole tips, simultaneously into corn oil cooled to 13°C. The resulting capsules were 3 mm in diameter.

### Example 2

Triple structure capsules were prepared in the same manner as in Example 1, except for replacing lidocaine to isosorbide dinitrate, PEG600 to Polyethylene glycol 2000 (PEG2000), and the fatty acid triglyceride having a melting point of 25°C to a fatty acid triglyceride having a melting point of 35°C in Example 1. The resulting capsules were 5 mm in diameter.

### Comparative Example 1

### Tablet

The following components were mixed uniformly in the following compositional ratios (% by weight) and then molded into tablets each having a weight of 70 mg.

| Component | part by weight |
|---|---|
| Lidocaine | 60 parts by weight |
| Lactose | 40 parts by weight |
| Corn starch | 75 parts by weight |
| Sodium caseinate | 6 parts by weight |
| Gelatin | 6 parts by weight |
| Cellulose | 115 parts by weight |
| Silicon dioxide | 3 parts by weight |
| Sucrose fatty acid ester | 6 parts by weight |

### Comparative Example 2

### Liniment

A liniment was prepared by uniformly mixing the following components in the following compositional ratios (% by weight).

| Component | part by weight |
|---|---|
| Lidocaine | 1 part by weight |
| Polyethylene glycol 4000 (PEG4000) | 20 parts by weight |
| Polyethylene glycol 400 (PEG400) | 10 parts by weight |
| Cera flava | 7 parts by weight |
| Cetanol | 10 parts by weight |
| Polysorbate 80 | 1 part by weight |

### Example 3

As to controlled releasability in dissolution in the mouth, a test was carried out as follows.

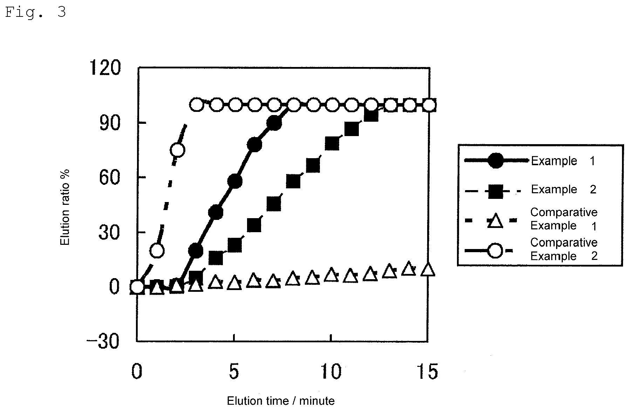

Paddle method of the elution test methods provided in the Japanese Pharmacopeia was used. 20 parts by weight of the capsules obtained in the above-described examples were added to 80 parts by weight of test solution A prepared by dissolving 0.1 parts by weight of sodium dodecyl sulfate in 100 parts by weight of a phosphate buffer (0.1 M, pH 6.8), and the solution was extracted in an appropriate amount every one minute. Each extract was diluted appropriately and an eluted amount was measured by high performance liquid chromatography. The result is shown in Fig. 3.

As is apparent from the result of Example 3, it shows that effective delay was observed in initial elution and medical agents were released in a pseudo-zero-order reaction in Examples 1 and 2. On the other hand, it shows that elution occurred too early in Comparative Example 1 and elution hardly occurred in Comparative Example 2.

### INDUSTRIAL APPLICABILITY

The oral medical composition of the present invention and the oral medical capsule containing the same as its content, which are medical agents in a form such that efficacy is exhibited when being licked in the oral cavity like a candy. Thus they can be effectively used especially for anesthesia or preliminary anesthesia in the oral cavity.

### BRIEF DESCRIPTION OF THE DRAWINGS

[Fig. 1] Fig. 1 is a schematic sectional view illustrating a capsule formulation of the present invention.
[Fig. 2] Fig. 2 is a schematic vertical sectional view illustrating one embodiment of a nozzle part of a manufacturing apparatus suitable for producing a seamless capsule formulation by the present invention.
[Fig. 3] Fig. 3 is a graph chart showing the result of the controlled releasability test of Example 3.

### DESCRIPTION OF SYMBOLS

1: Inner nozzle (first nozzle), 2: intermediate nozzle (second nozzle), 3: outer nozzle (third nozzle), 4: oral medical composition, 5: intermediate layer solution, 6: capsule film solution, 7: oral medical capsule, 8: cooling liquid, 10: capsule film, 11: capsule content, 12: intermediate layer, 20: oral medical capsule.

## Claims

1. An oral seamless capsule comprising a capsule film, a content enclosed in the film, and an intermediate layer intervening between the content and the capsule film, wherein the content is an oral drug composition comprising
(a) a drug being a local anesthetic that is low in solubility in both water and oil, but is soluble in a polyethylene glycol,
(b) a polyethylene glycol that is solid at temperatures of 40°C or lower,
(c) an oily or aqueous auxiliary agent, and
wherein the intermediate layer is made of fat having a melting point of 40°C or lower.

2. The oral medical capsule according to claim 1, wherein the auxiliary agent (c) is a freshener, an anti-inflammatory agent, a local anesthetic, or a mixture thereof.

3. The oral medical capsule according to claim 1, wherein the medical agent (a) is contained in an amount of 0.01 to 40% by weight in the oral medical composition.

## Patentansprüche

1. Orale nahtlose Kapsel, umfassend einen Kapselfilm, einen in dem Film eingeschlossenen Inhalt und eine Zwischenschicht, die zwischen dem Inhalt und dem Kapselfilm liegt, wobei der Inhalt eine orale pharmazeutische Zusammensetzung ist, umfassend:
(a) einen Wirkstoff, bei dem es sich um ein Lokalanästhetikum handelt, das sowohl in Wasser als auch in Öl eine geringe Löslichkeit aufweist, aber in einem Polyethylenglycol löslich ist;
(b) ein Polyethylenglycol, das bei Temperaturen von 40 °C oder darunter fest ist;
(c) ein öliges oder wässriges Hilfsmittel; und
wobei die Zwischenschicht aus Fett mit einem Schmelzpunkt von 40 °C oder darunter besteht.

2. Orale medizinische Kapsel gemäß Anspruch 1, wobei das Hilfsmittel (c) ein Erfrischungsmittel, ein entzündungshemmendes Mittel, ein Lokalanästhetikum oder ein Gemisch davon ist.

3. Orale medizinische Kapsel gemäß Anspruch 1, wobei das medizinische Mittel (a) in einer Menge von 0,01 bis 40 Gew.-% in der oralen medizinischen Zusammensetzung enthalten ist.

## Revendications

1. Capsule orale sans soudure comprenant un film de capsule, un contenu enfermé dans le film, et une couche intermédiaire interposée entre le contenu et le film de capsule, dans laquelle le contenu est une composition médicamenteuse orale comprenant :
(a) un médicament qui est un anesthésiant local de faible solubilité dans l'eau et dans l'huile, mais qui est soluble dans un polyéthylèneglycol,
(b) un polyéthylèneglycol qui est solide à des températures de 40 °C ou moins,
(c) un agent auxiliaire huileux ou aqueux, et
dans laquelle la couche intermédiaire est composée d'une graisse ayant un point de fusion de 40 °C ou moins.

2. Capsule médicale orale selon la revendication 1, dans laquelle l'agent auxiliaire (c) est un agent rafraîchissant, un agent anti-inflammatoire, un anesthésiant local, ou un de leurs mélanges.

3. Capsule médicale orale selon la revendication 1, dans laquelle l'agent médical (a) est présent dans une quantité de 0,01 à 40 % en poids de la composition médicale orale.
